# EUROPEAN PATENT APPLICATION

(11) **EP 2 889 035 A1**
(43) Date of publication of application: **01.07.2015**
(21) Application number: 13821780.7
(22) Date of filing: 26.08.2013
(51) Int. Cl.: A61K 31/7016, A23L 1/30, A61P 35/00

(54) **ANTI-TUMOR AGENT**

(30) Priority: 24.08.2012 WO PCT/JP2012/071414
(71) Applicant: Kakui Co., Ltd., Kagoshima 890-0081 (JP)
(72) Inventor: IWAMOTO, Masataka, Kagoshima-shi Kagoshima 890-0081 (JP)
(74) Representative: Schrell, Andreas
(86) International application number: PCT/JP2013/072686
(87) International publication number: WO 2014/030763

(57) **Abstract**

The objective of the present invention is to provide a highly safe antitumor agent having excellent antitumor activity and few side effects. The antitumor agent of the present invention is characterized in that it contains cellobiose as an active ingredient.

## Description

### Technical Field

The present invention relates to an antitumor agent.

### Background Art

Many studies have been conventionally conducted to develop therapeutic methods appropriate for treatment of cancer and alleviation of its associated symptoms. As cancer therapies, namely, three major therapies are adequately employed: chemotherapy, radiation therapy, and surgical therapy. Radiation therapy has several side effects, including acute effects and late-phase responses. Specifically, radiation therapy can cause various problems such as malaise, anorexia, skin disorders such as erythema, bleeding from digestive organs, decreases in white blood count, and alopecia, due to the damage to normal cells in addition to cancer cells caused by radiation. Surgical therapy is mentally and physically demanding for patients, and may cause surgical sequelae or complications. Surgical therapy is also problematic in that it cannot be applied when cancer occurs at a site that is difficult to surgically resect.

Meanwhile, in the field of chemotherapy, effective antitumor agents against cancer cells have been discovered, such as antibiotics, antimetabolites, alkylating agents, and hormone preparations. However, these antitumor agents not only attack cancer cells, but also affect on normal cells. Hence, a problem occurs in that these antitumor agents induce side effects (e.g., vomiting, nausea, anorexia, and alopecia), thereby significantly decreasing patients' quality of life (QOL). Therefore, the development of a novel and highly safe antitumor agent with fewer side effects is desirable.

Patent Document 1 discloses an antitumor agent comprising an acidic xylo-oligosaccharide with an uronic acid residue in a xylo-oligosaccharide molecule and an extract from *Lyophyllum decastes,* as active ingredients. Moreover, Patent Document 2 discloses an antitumor substance comprising a sugar ester of a monosaccharide or a disaccharide and a fatty acid. However, these antitumor agents have insufficient anti-tumor effects and there is room for their improvement.

Patent Document 3 discloses an agent for activating enteric bacteria, which comprises a cellooligosaccharide, such as cellobiose, as an active ingredient. According to this invention, the cellooligosaccharide causes a selective increase in useful enteric bacteria, which allows suppression of an increase in harmful bacteria. However, cellooligosaccharides such as cellobiose have never conventionally been examined for antitumor activity.

Also, Patent Document 4 discloses the cytotoxic effects of pharmaceutical products containing cellobiose on cancer cells. However, as described later, the substance used in Patent Document 4 is an extract from a plant, which is an impure sample of cellobiose. This can be clearly understood also from the NMR data described in Patent Document 4. Moreover, a test concerning the survival rate of cancer cells described in Patent Document 4 demonstrated that the extract had cytotoxic effects. This also suggests that the extract is an impure sample of cellobiose.

### Prior Art Documents

### Patent Documents

Patent Document 1: JP Patent Publication (Kokai) No. 2008-208093 A
Patent Document 2: JP Patent Publication (Kokai) No. 2002-179577 A
Patent Document 3: JP Patent Publication (Kokai) No. 2007-330177 A
Patent Document 4: International Publication WO2011/110190

### Summary of the Invention

### Problem to be Solved by the Invention

In view of the above conventional circumstances, an object of the present invention is to provide a highly safe antitumor agent having excellent antitumor activity and few side effects.

### Means for Solving the Problem

As a result of intensive studies to achieve the above objective, the present inventors have discovered that cellobiose, which is a kind of oligosaccharide, suppresses the development of cancer that is induced by carcinogens, and thus have completed the present invention. Specifically, the present invention is as summarized as follows.
(1) An antitumor agent, comprising cellobiose as an active ingredient.
(2) A pharmaceutical product, containing the antitumor agent according to (1) above.
(3) A food, containing the antitumor agent according to (1) above.

### Effects of the Invention

According to the present invention, a highly safe antitumor agent having excellent antitumor activity as well as pharmaceutical products and foods produced using the same can be obtained.

### Brief Description of the Drawings

Fig. 1 shows a graph showing changes in the body weight of animals of each group in Examples.
Fig. 2 shows a graph showing changes in the number of animals having developed tumors of each group in Examples.
Fig. 3 shows a graph showing changes in the tumor volume estimate of animals of each group in Examples.
Fig. 4 shows a graph showing the wet weights of tumors of animals of each group in Examples.
Fig. 5 shows the ¹³C NMR spectrum of the test substance (cellobiose) in the reference example.
Fig. 6 shows the ¹H NMR spectrum of the test substance (cellobiose) in the reference example.
Fig. 7 shows the infrared spectrum of the test substance (cellobiose) in the reference example.
Fig. 8 shows a graph showing the cytotoxic activity of the test substance (cellobiose) on HeLa.
Fig. 9 shows a graph showing the cytotoxic activity of the test substance (cellobiose) on HaCaT.
Fig. 10 shows a graph showing the cytotoxic activity of the test substance (cellobiose) on Hep-G2.
Fig. 11 shows a graph showing changes in survival rate with respect to the concentration of dexamethasone.

### Embodiments for Carrying out the Invention

Hereafter, the present invention is described in greater detail.

The antitumor agent according to the present invention is characterized by containing cellobiose as an active ingredient. Cellobiose is a disaccharide, consisting of two glucose molecules linked by a β 1,4 bond. The chemical formula thereof is C₁₂H₂₂O₁₁. Cellobiose can be appropriately produced by a method that involves degrading a cellulose-based substance with cellulase, a method that involves performing condensation or glycosyltransfer of monosaccharides of glucose (sugar), or derivatives thereof, or a method that involves synthesizing it from sucrose, for example.

Both vegetable and animal substances are applicable as cellulose-based substances which are degraded by cellulase. Specifically, natural product-derived fibrous substances contained in cotton, wood, bamboos, Kenaf (*Hibiscus cannabinus*), wheat (e.g., wheat, barley, and oats), rice, bacterial cellulose, and the like, regenerated cellulose produced by dissolving such a fibrous substance in a solvent, or cellulose derivatives produced via chemical treatment thereof can be used, for example. Any one type of these cellulose-based substances, or two or more types thereof can be used in combination. In particular, natural cellulose-based substances not dissolved or not subjected to chemical treatment are preferred since the thus obtained cellobiose contains neither solvent nor chemical substance harmful to human bodies.

As cellulase, the one having the activity to degrade cellulose is applicable. Examples of a cellulase enzyme source include cellulase-producing microorganisms themselves, a source prepared by purifying an enzyme that is secreted by a cellulase-producing microorganism, and a source prepared by formulating a purified enzyme together with an additive such as an excipient or a stabilizer. The types of additive in preparations are not particularly limited. The dosage form thereof may be any of powders, granules, and liquid.

An example of a method for enzymatic degradation using cellulase is a method that involves suspending a cellulose-based substance in an aqueous medium, adding cellulase to the suspension, and then heating the mixture while stirring or shaking in order to perform a saccharification reaction. The method for suspension, the method for stirring, the method for adding a cellulose-based substance and cellulase and the order of addition, and reaction conditions (e.g., concentrations thereof) in this case can be appropriately set in view of cellobiose yield and other factors. Conditions of the pH, the temperature and other conditions of the reaction solution may be conditions that do not allow cellulase deactivation. Specifically, when a reaction is performed under normal pressure, the temperature can range from 5°C to 95°C, and the pH can range from 1 to 11.

In a preferred example, cellobiose in the present invention can be obtained by enzymatic degradation of cotton fiber (absorbent cotton). This method is advantageous in that the processing time is extremely shorter than that of a method using woody raw material. The product obtained by the enzymatic degradation of cotton fiber contains about 75% cellobiose, 22% glucose, and 3% cellotriose, as confirmed by HPLC measurement.

The thus produced cellobiose can be subjected to purification treatment such as, enzyme removal, desalting, or decolorization, as necessary. Any known purification method can be used without particular limitation. Examples thereof include chromatography treatment, filtration treatment (e.g., microfiltration, ultrafiltration, and reverse osmosis membrane filtration), crystallization treatment, treatment using an ion exchange resin, and activated carbon treatment. These examples of treatment can be performed independently or multiple examples thereof can be performed in combination.

Cellobiose that is obtained as described above has antitumor activity against various types of cancer such as breast cancer, by which tumor development or tumor growth is suppressed. Thus, the cellobiose can be used as a pharmaceutical product. When an antitumor cellobiose agent is used as a pharmaceutical product, the form thereof is not particularly limited, and can be appropriately selected from various forms such as tablets, capsules, powders, granules, and drinkable preparations depending on the method of administration or conditions to be applied (e.g., such as tumor types, tumor shapes, and tumor sites). Specifically, an antitumor cellobiose agent can be directly used as a pharmaceutical product, or, it can be formulated into a pharmaceutical product in combination with a general additive such as a carrier, a diluent, or an excipient, as necessary.

The intake or the dosage of the antitumor agent according to the present invention is a therapeutically effective amount and differs depending on tumor types and the like. In the case of an adult with a body weight of 60 kg, the dosage of cellobiose per day is generally 240 mg or more, and is preferably 2400 mg or more. The upper limit of the dosage is not particularly limited, and is preferably less than 4800 mg. Also, the antitumor agent of the present invention can also be used in combination with a known antitumor agent or another therapeutic agent.

Furthermore, the antitumor cellobiose agent according to the present invention can be contained in general foods and then such foods can be used as functional foods having antitumor activity. Moreover, cellobiose can be encapsulated using gelatin or the like and then the capsule may be used as a supplement, or may be incorporated into beverages, sweets, gum, candies, or the like.

### Examples

Next, the present invention is described in greater detail on the basis of examples. However, the following examples are simply given for illustrative purposes and the present invention is not limited by these examples.

### (Examples)

### 1. Test Objective

The antitumor effects of cellobiose were examined using a rat DMBA-induced breast cancer model.

### 2. Standards applied

This test was conducted as directed under the following laws, standards, and guidelines.
- Act on Welfare and Management of Animals [Act No. 105, October 1, 1973 (August 30, 2011, final revision: Act No. 105)
- Standards relating to the care, management, and alleviating pain, etc. of experimental animals (Notification No. 88 of the Ministry of the Environment, April 28, 2005)
- Guidelines relating to methods for the culling of animals [Notification No. 40 of the Prime Minister's Office, July 4, 1995 (November 12, 2007, partial amendment: Notification No. 105 of the Ministry of the Environment)]

In addition, the test was conducted after the animal experiment plan defined by the Institutional Animal Experimentation Committee had been deliberated and approved.

### 3. Summary of test

The antitumor effects of cellobiose were examined using the rat DMBA-induced breast cancer model. Specifically, 7-week-old female rats were grouped using body weight as an index, and then 7,12-dimethylbenz[a]anthracene (DMBA) (20 mg/mL/rat) was forcibly administered orally. Two doses of the test substance (4 and 40 mg/kg/day) were forcibly administered orally once a day from the day after grouping for 12 consecutive weeks.

As a result, both in the low-dose and high-dose groups, tumors developed later than in the control group. The high-dose group exhibited tumor weights lower than the control group, but this was not statistically significant. The results suggested that the test substance, cellobiose, suppresses the development of breast cancer that is induced by carcinogenic DMBA.

### 4. Test materials and tools

### (1) Test substance and medium

i) Test substance
   Name: cellobiose
   Nature: powder
   Storage conditions: low-temperature storage
   Cautions for handling: the test substance was stored in a sealed case after use to avoid humidity due to its high hygroscopicity.
ii) Distilled water
   Name: water for injection
   Storage conditions: room temperature
   Manufacturer: Otsuka Pharmaceutical Factory Inc.
   Cautions for handling: no particular cautions.
iii) CMC
   Name: sodium carboxy methylcellulose
   Storage conditions: room temperature
   Manufacturer: Kanto Chemical Co., Inc.
   Cautions for handling: no particular cautions.

### (2) Reagents used

The following reagents were used as carcinogens.
i) DMBA
   Name: 7,12-dimethylbenz[a]anthracene
   Storage conditions: room temperature
   Manufacturer: SIGMA
   Cautions for handling: masks, safety eyeglasses, and gloves were used in order to avoid inhalation and adhesion to skin.
ii) Sesame oil
   Name: sesame oil
   Storage conditions: room temperature
   Manufacturer: SIGMA
   Cautions for handling: no particular cautions

### (3) Animals used

Fifty-two 6-week-old female rats, Crl: CD (SD) (SPF), were purchased from Charles River Laboratories, Japan Inc. on March 28, 2012 (Number of rats ordered: 50).

The animals were individually recognized using the ear punching method on the day of delivery, and then they were conditioned from the day of delivery to the day of grouping. General conditions were observed every day. Animals for which no abnormalities had been observed in terms of general conditions and changes in body weight were selected and used. A card containing test number, gender, and individual recognition number (ear punch number) was attached to each cage before grouping. After grouping, group and animal numbers were added thereto.

### (4) Feeding procedure

Throughout conditioning, feeding, and experimental periods, animals were housed individually in 1 compartment (255W x 185D x 200H, mm) of a stainless wire mesh double cage provided in a movable stainless rack (1790W x 470D x 1650H, mm) under certain environmental conditions (temperature: 22±3°C, humidity: 50±20%, 12 hours of illumination (8:00 to 20:00), and ventilator rate: 13 to 17 times/hour). Regarding feedstuff, animals were fed *ad libitum* with the solid feedstuff CRF-1 (Oriental Yeast Co., Ltd.) using a stainless feeder for solid feedstuffs. Animals were also fed *ad libitum* with tap water using a polysulfone waterer (with a stainless-steel tube end).

### 5. Test method

### (1) Grouping

Animals, for which no abnormalities had been confirmed as a result of observation of general conditions during the quarantine and conditioning periods, were selected and then grouped by the stratified continuous randomization method using body weight as an index based on the following table of group composition (Table 1). The remaining animals were excluded from the test groups on the day of grouping and then disposed of after excessive euthanization with ether.

**Table 1**

| Table of group composition | | | | |
|---|---|---|---|---|
| Group | Dosage (mg/kg/day) | Fluid volume administered (mL/kg/day) | Number of animals (rats) | Animal number |
| Control | 0^{a)} | 5 | 15 | 001-015 |
| Low dose | 4 | 5 | 15 | 101-115 |
| High dose | 40 | 5 | 15 | 201-215 |

| | | | | |
|---|---|---|---|---|
| a) Medium was administered. | | | | |

### (2) Setting of animals for monitoring

Three animals, for which no abnormality had been observed during quarantine and conditioning periods, were selected from those remaining after grouping, in ascending order of individual recognition numbers, for monitoring of the feeding environment. Thus the animals for minoring were determined. These animals for monitoring were fed within the same animal room as that for the test groups until the final autopsy date of the test groups.

### (3) Administration of carcinogen

### i) Preparation of carcinogen

DMBA was weighed to the required amount using an electronic even Sartorius Laboratory balance (SARTORIUS K.K.). The weighed DMBA was added to a beaker containing sesame oil, and then the solution was stirred using a mighty magnetic stirrer (Koike precision instruments) until the DMBA was completely dissolved. After dissolution, the solution was transferred to a volumetric graduated cylinder and then diluted to a concentration of 20 mg/mL. Upon preparation, countermeasures were taken against chemical hazards to avoid inhalation and adhesion to skin by wearing masks, safety eyeglasses, and gloves. Preparation was performed before use. Any carcinogen remaining after administration was treated as medical waste.

### ii) Administration of carcinogen

The carcinogen was administered to each animal after grouping. Specifically, the carcinogen was forcibly administered orally at a dosage of 1 mL/animal using sonde for oral administration (disposable sonde for oral administration, FUCHIGAMI) and glass syringes (disposable syringes, TERUMO Corporation). Administration was performed using masks, safety eyeglasses and gloves in order to avoid inhalation and adhesion to skin.

### (4) Administration of test substance

### i) Preparation of test substance solution to be administered

Medium was prepared as follows. CMC was weighed to the required amount using an electronic even Sartorius Laboratory balance (SARTORIUS K.K.). Distilled water in a beaker was gradually added to the weighed CMC while stirring the solution using a mighty magnetic stirrer (Koike precision instruments). The solution was transferred to a volumetric graduated cylinder and then diluted to 0.5% (W/V). The thus prepared medium was stored under refrigeration for up to 9 days including the day of preparation, and then used.

Next, the test substance was weighed to the required amount using an electronic Sartorius Analytic balance (SARTORIUS K.K.), and then dissolved in the medium. The resultant was transferred to a volumetric graduated cylinder, and then diluted to concentrations of 0.8 mg/mL and 8.0 mg/mL. Preparation was performed before use. The solution of the test substance (to be administered) that had remained after administration was diluted with a large volume of tap water and then discarded.

### ii) Administration of test substance

Administration of the test substance began the day after grouping (designated as day 1). The test substance was forcibly administered orally once a day for 12 consecutive weeks.

Upon administration, the test substance solution was forcibly administered orally at 5 mL/kg/day using sonde for oral administration (disposable sonde for oral administration, FUCHIGAMI) and glass syringes (disposable syringes, TERUMO Corporation) according to the table of group composition. The fluid volume administered was calculated based on the body weight measured on the day closest to the date of administration.

### (5) Observation of general conditions and measurement of body weight

After administration of the carcinogen, the general conditions were observed every day. Body weight was measured once a week.

### (6) Calculation of tumor volume estimate during the period of test substance administration

When the development of tumors was confirmed, the number of tumors developed in each animal was measured, and then the tumor volume estimate (mm³) of each tumor was calculated once a week. In addition, the tumor volume estimate (mm³) of each tumor was found by shaving the hair around the relevant tumor, measuring the tumor size (major axis "a" and minor axis "b" (mm)) using a vernier caliper, calculating according to the following formula, and then finding the sum of tumor volume estimates of the tumors.
Tumor volume estimate (mm³) of each tumor = 0.5 x a x b²

### (7) Collection of feces

Feces were collected on the final day of the administration period. In the morning on the day of collection, animals were each transferred from the double cages to breeding cages. The animals were left to stand for 1 hour, feces excreted in the breeding cage of each animal were collected, and then stored in a deep freezer set at -80°C.

### (8) Autopsy

Autopsy was performed the day after the final day of the administration period. Animals underwent laparotomy under ether anaesthesia, and were then euthanized by bleeding from the abdominal aorta. Subsequently, all tumors of each animal were extracted, and then their wet weights were measured using an electronic balance (TX323L, Shimadzu Corporation). Tumors were discarded after measurement of the wet weights.

### (9) Statistical processing

The mean and standard errors of the thus obtained numerical values (body weights, tumor volume estimates, and extracted tumor weights upon autopsy) were calculated for each group.

Regarding the significant difference between groups, homoscedasticity between the groups was tested using the Bartlett method. When the result of this was homoscedastic, a one-way analysis of variance was further conducted. When the result of this was statistically significant, mean values were compared by the Tukey method. When the result of this was heteroscedastic, the Kruskal-Wallis H test was conducted. When the result of this was statistically significant, mean ranks were compared by the Tukey method. For the Bartlett method, the one-way analysis of variance, and the Kruskal-Wallis H test, the significance level (risk) was determined to be 5%. For the Tukey method, the significance levels (risk) were determined to be 5% and 1%.

### 6. Test results

### (1) General conditions and changes in body weight

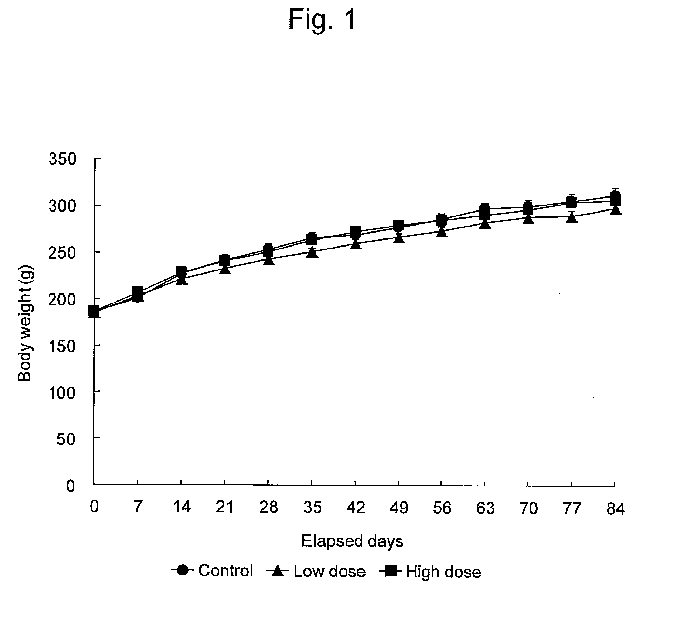

Fig. 1 shows changes in the body weight of animals of each group. The control group exhibited increases in body weight throughout the test period. The low-dose group and the high-dose group exhibited very similar changes to the control group. No significant difference between groups was observed.

Regarding general conditions, one death was confirmed on day 8 in the control group (animal number: 007). In the low-dose group, one death was confirmed on day 4 (animal number: 106). In the high-dose group, one death was confirmed on day 76 (animal number: 209).

### (2) Changes in the number of animals having developed tumors

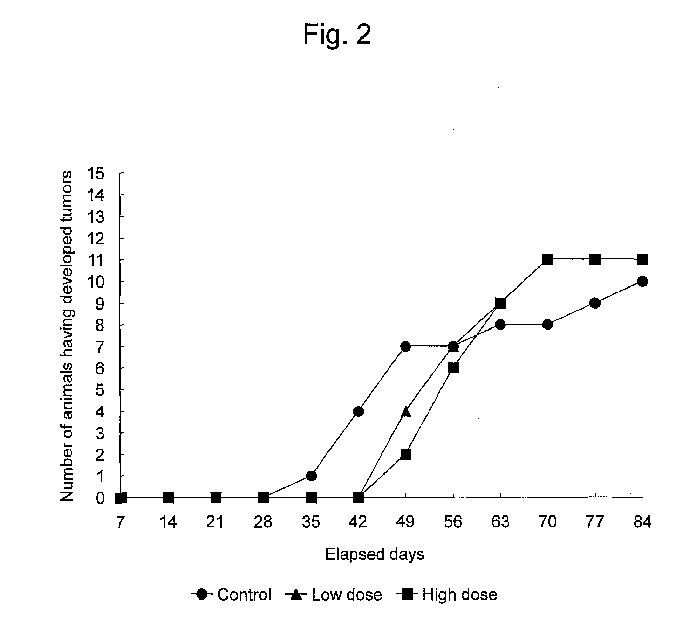

Fig. 2 shows changes in the number of animals having developed tumors of each group. In the control group, the number of animals for which the development of tumors had been confirmed on day 35 was 1. Thereafter, the number of animals for which the development of tumors had been confirmed was found to increase and was 10 on day 84. In the low-dose group, no development of tumors was confirmed until day 42. On day 49, the number of animals for which the development of tumors was confirmed was 4. Thereafter, the number of animals for which the development of tumors was confirmed was found to increase and was 11 on day 84. In the high-dose group, no development of tumors was confirmed until day 42. On day 49, the number of animals for which the development of tumors had been confirmed was 2. Thereafter, the number of animals for which the development of tumors had been confirmed was found to increase and was 11 on day 84.

### (3) Changes in tumor volume estimate

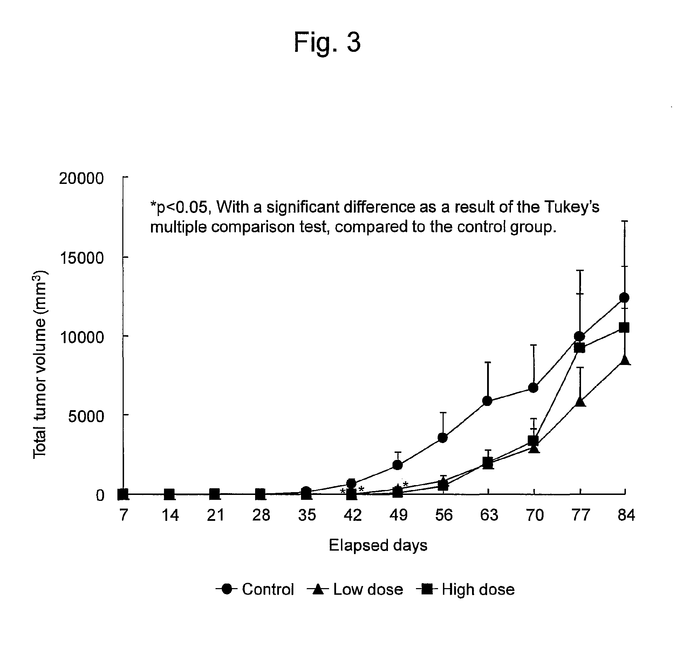

Fig. 3 shows changes in tumor volume estimate of each group. In the control group, the development of tumors was confirmed from day 35 (after 5 weeks). Thereafter, new development of tumors was confirmed and the volume of each tumor was also found to increase. In the low-dose group, the development of tumors was confirmed from day 49 (after 7 weeks). Thereafter, new development of tumors was confirmed and the volume of each tumor was found to increase. On day 42, the values were significantly lower than those of the control group. In the high-dose group, the development of tumors was confirmed from day 49 (after 7 weeks). Thereafter, new development of tumors was confirmed and the volume of each tumor was found to increase. On days 42 and 49, the values were significantly lower than those of the control group.

### (4) Wet weight of tumor

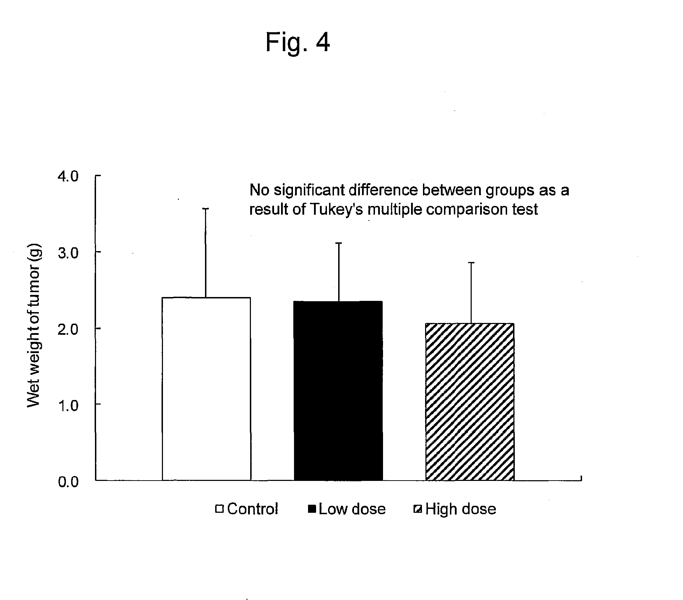

Fig. 4 shows the wet weights of the tumors of each group. In the low-dose group, the wet weights were very similar to those of the control group. The high-dose group exhibited lower wet weights than those of the control group, but this was not statistically significant.

### 7. Discussion and general overview

The tumor development in both the low-dose group and the high-dose group was later than in the control group. The high-dose group exhibited lower tumor weights compared to the control group, but this was not statistically significant. These results suggested that the test substance, cellobiose, suppresses the development of breast cancer that is induced by carcinogenic DMBA.

### (Reference example 1)

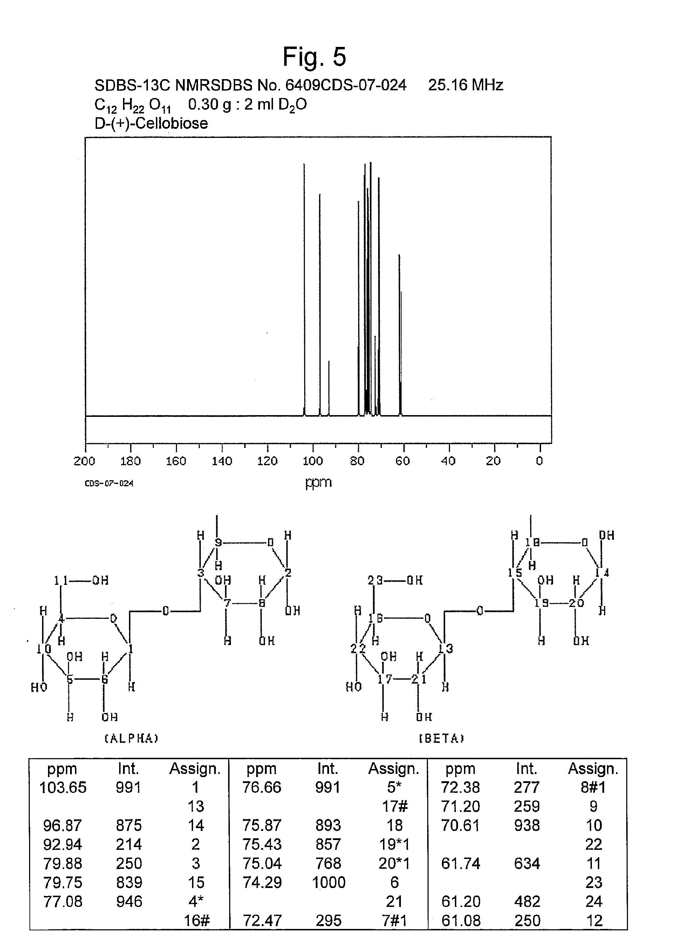

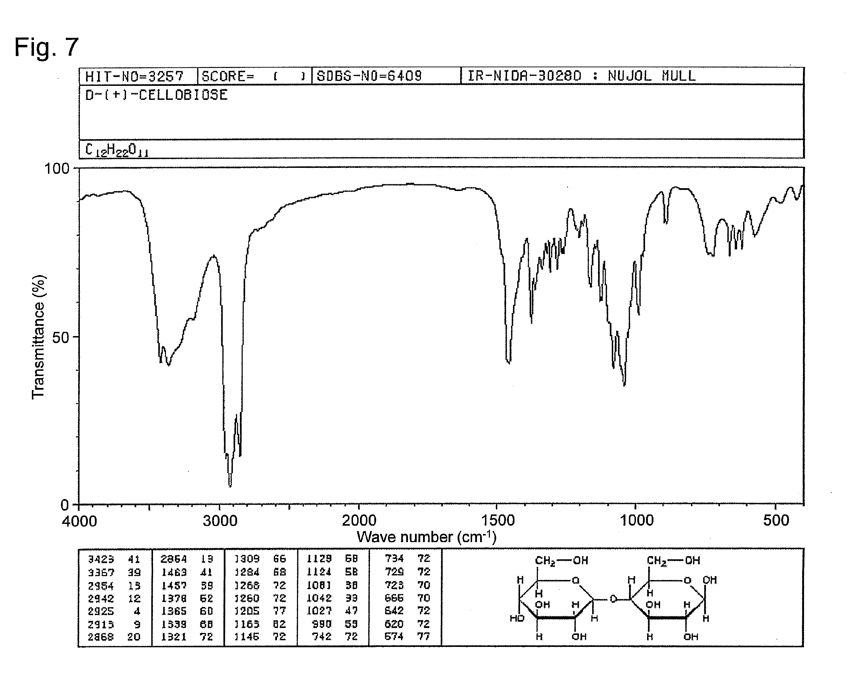

¹³C NMR, ¹H NMR, and IR measurements were performed for cellobiose. The thus obtained NMR spectrum and infrared spectrum are shown in Fig. 5 to Fig. 7.

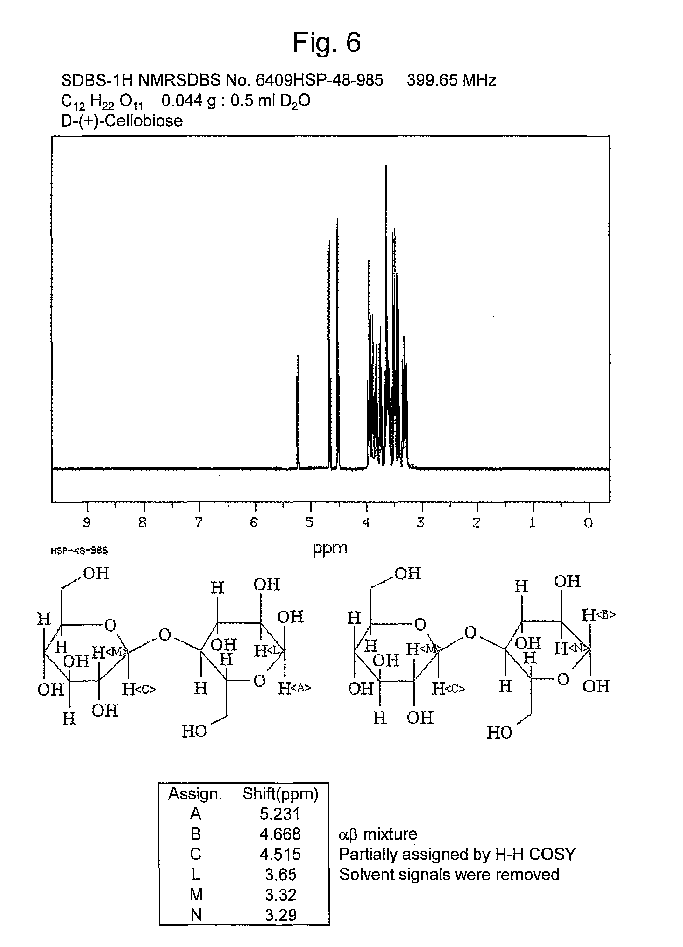

As is clear from comparison between Fig. 6 and the above Fig. 4 (Patent Document 4) for NMR chart, peaks peculiar to cellobiose were confirmed in both charts. However, in the NMR chart in Fig. 4, a peak was also confirmed in another position. The results revealed that whereas the cellobioses shown in Fig. 5 to Fig. 7 are high-purity cellobioses, the one described in Patent Document 4 is an impure sample of cellobiose.

### (Reference example 2)

Cell survival rate was tested using cellobiose by the following method.

### <Test for examining the effects of the test substance (cellobiose) on cell survival>

The presence or the absence of the selective cytotoxic activity of the test substance (cellobiose) was examined on various established cell lines.

### 1. Test materials

### (1) Test substance

Name: cellobiose
Storage method: refrigeration, in the dark, dehumidification

### (2) Cells used herein

Cell species: HeLa (human cervical cancer-derived cell line), HaCaT (human-derived immortalized keratinocyte) and Hep-G2 (human liver cancer-derived cell line)

### (3) Media used herein

Medium name: medium specialized for HeLa, medium specialized for HaCaT, and medium specialized for Hep-G2

### (4) Reagents used herein (for XTT assay)

Reagent name: Cell proliferation kit (XTT)
Manufacturer name: COSMO BIO co., ltd.
Catalog No.: 20-300-1000

### 2. Test method

### (Thawing and passage culture of cells)

(1) HeLa, HaCaT, and Hep-G2 cells were thawed and then cultured in various specialized media.
(2) Cells were passaged 2 to 3 times.
(3) Cells that had recovered their growth capacity were used for the test.

### (Cell culture and XTT assay)

(1) HeLa, HaCaT, and Hep-G2 cells were each seeded in 24 wells of each of two 96-well plates at 1 x 10⁴ cells/100 µL/well.
(2) Cells were cultured for 24 hours in a CO₂ incubator.
(3) The test substance was added at 6 serial concentrations of 0 (control), 0.001, 0.01, 0.1, 1, and 10 mg/mL (N=4).
(4) After addition, cells were cultured for 3 hours in the CO₂ incubator.
(5) After culture, photographs of the cells were taken.
(6) After taking photographs, the medium containing the test substance was removed from one of the 96-well plates where various cells had been cultured, and thus exchanged with a general medium.
(7) 50 µL of an XTT reagent was added.
(8) After addition, cells were cultured for 3 hours in a CO₂ incubator.
(9) Absorbance was measured at 450 nm to 500 nm (ref.: 630 nm to 690 nm).

### (Confirmation of the performance of cell proliferation kit (XTT))

(1) HeLa cells were seeded in 16 wells of a 96-well plate at 1 x 10⁴ cells/100µL/well.
(2) Cells were cultured for 24 hours in a CO₂ incubator.
(3) Dexamethasone was added at 3 serial concentrations of 0 (control), 10, 100, and 500 µM (N=4).
(4) After addition, cells were cultured for 3 hours in the CO₂ incubator.
(5) 50 µL of an XTT reagent was added.
(6) After addition, cells were cultured for 3 hours in the CO₂ incubator.
(7) Absorbance was measured at 450 nm to 500 nm (ref.: 630 nm to 690 nm).

### 3. Test results

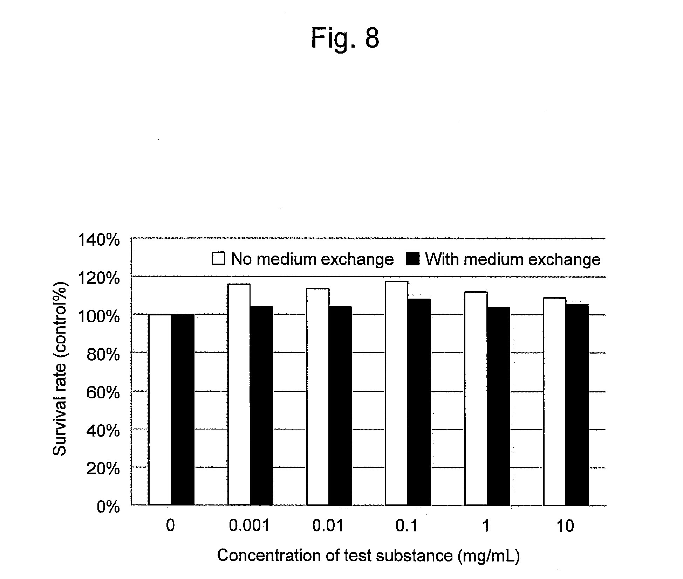

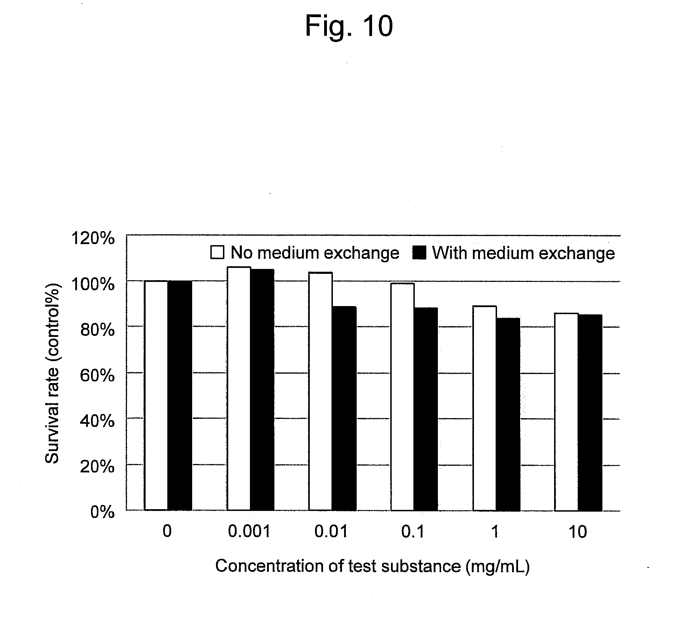

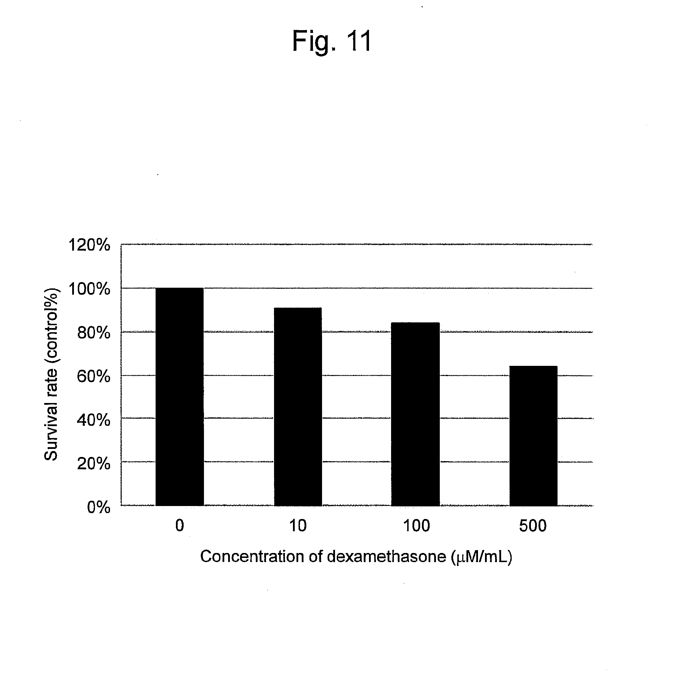

Fig. 8 to Fig. 10 show the results of XTT assay for HeLa, HaCaT, and Hep-G2. As is clear from Fig. 8 to Fig. 10, almost no change in survival rate was confirmed for HeLa and HaCaT even at the test substance concentration of 10 mg/mL, compared to the control. In addition, a maximum of about a 15% decrease was observed in the survival rate of the Hep-G2 group, to which the test substance with a high concentration had been administered. Moreover, to confirm the performance of the kit used herein, an assay was performed using HeLa and an apoptosis-inducing agent, dexamethasone. The results are shown in Fig. 11. A decrease in survival rate of about 40% was observed in the cells subjected to assay with 500 µM dexamethasone. It was thus confirmed that the kit was performing adequately.

Furthermore, as shown in photomicrographs, no influence of other factors such as an increase in dead cells or cell morphological changes, was observed in HeLa, HaCaT, or Hep-G2 at any concentration of the test substance, compared to the cells before addition of the test substance.

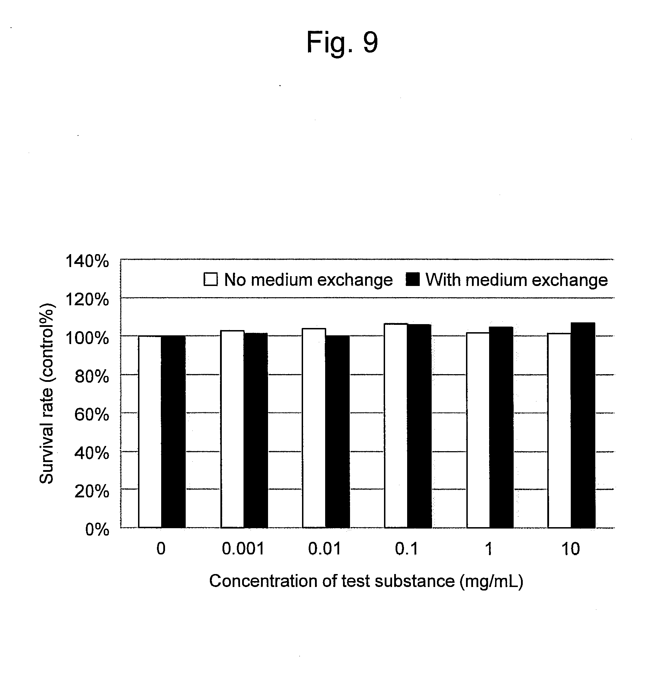

Fig. 8 to Fig. 10 show that high-purity cellobiose; that is the substance of interest of the present invention, has no cytotoxic effect on cancer cells. On the other hand, Fig. 9 (Patent Document 4) shows that a compound has a cytotoxic effect on cancer cells. Accordingly, it was revealed that the compound described in Patent Document 4 is an impure sample of cellobiose, and an ingredient having a cytotoxic effect on cancer cells is the one other than cellobiose. Therefore, Patent Document 4 does not disclose any technical idea concerning the antitumor effects of cellobiose. In addition, as shown in Fig. 8 to Fig. 10, the fact that high-purity cellobiose has no direct cytotoxic effect on cancer cells is consistent with the fact that cellobiose suppresses the development of cancer that is induced by a carcinogen, as proven in examples above. Specifically, in the present invention, cellobiose does not directly act on cells, but acts on the immune system of an organism to enhance the immunity, and as a result exhibits antitumor effects.

All publications, patents, and patent applications cited herein are incorporated herein by reference in their entirety.

## Claims

1. An antitumor agent, comprising cellobiose as an active ingredient.

2. A pharmaceutical product, containing the antitumor agent according to claim 1.

3. A food, containing the antitumor agent according to claim 1.
